# EUROPÄISCHE PATENTSCHRIFT

(11) **EP 2 193 757 B1**
(45) Veröffentlichungstag und Bekanntmachung des Hinweises auf die Patenterteilung: **21.03.2018**
(21) Anmeldenummer: 10002986.7
(22) Anmeldetag: 18.04.2003
(51) Int. Cl.: A61C 1/14, A61C 1/18, A61B 17/16, A61C 3/02, A61C 1/05, F16C 35/073, F16C 19/54

(54) **Drehmomentübertragungseinrichtung für ein chirurgisches oder dentales, rotierendes Werkzeug**
Torque transmission device for a surgical or dental rotating instrument
Dispositif de transmission de couple d'un instrument rotatif chirurgical ou dentaire

(30) Priorität: 18.04.2002 AT 6032002
(43) Veröffentlichungstag der Anmeldung: 09.06.2010
(62) Teilanmeldung aus: 03720005.2
(73) Patentinhaber: W & H Dentalwerk Bürmoos GmbH, 5111 Bürmoos (AT)
(72) Erfinder: Helfenbein, Gerald, 5133 Gilgenberg (AT)
(74) Vertreter: Patentanwälte Barger, Piso & Partner

(56) Entgegenhaltungen:
- WO-A2-98/05261
- DE-A1- 19 918 638
- DE-U1- 9 101 385
- US-A- 6 068 632

## Beschreibung

Die Erfindung betrifft ein Handstück für ein chirurgisches oder dentales, in den Kopf des Handstückes durch eine Einstecköffnung in eine Werkzeugaufnahme einsteckbares, mittels zweier, axialen Abstand voneinander aufweisenden Lager rotierend gelagertes Werkzeug mit einem im Bereich des Lagers, das der Einstecköffnung benachbart ist, vorgesehenen Bereich zur formschlüssigen Drehmomentübertragung auf das Werkzeug.

Ein nicht gattungsgemäßes Handstück ohne Zufuhrröhrchen, somit ohne Möglichkeit der Innenzufuhr von Kühlmitttel bzw. Spülmittel durch das Werkzeug, und mit anderer Drehmomentübertragung ist aus der AT 375 011 B bekannt, bei dem, mit dem Werkzeug unverlierbar verbunden, ein Zahnrad, ein Lager für das Werkzeug und ein Teil des Kopfgehäuses als Einsatzteil vorgesehen ist. Der Grund für diese Maßnahmen liegt im Wunsch der möglichsten Miniaturisierung des Kopfes des Handstückes. Auf eine Werkzeugaufnahme wird aus diesem Grund verzichtet und dafür der hohe Aufwand mit dem Einsatzteil in Kauf genommen. Dass dieser, auf den restlichen Kopf (und das zweite Lager!) exakt abgestimmte Einsatzteil im Vergleich zum Werkzeug extrem teuer und wegen des Lagers extrem schlecht zu reinigen und zu desinfizieren ist, liegt auf der Hand.

Ein anderes, ebenfalls nicht gattungsgemäßes Handstück ohne Zufuhrröhrchen, mit einer Drehmomentübertragung über eine Art Klemmring ist aus der US 5,542,846 A bekannt. Dabei wird der Klemmring drehfest in einer Führungshülse gehalten und durch eine Feder schräg gestellt, wodurch er mittels Reibschluß den Schaft des Werkzeuges mitdreht.

Die WO 98/05261 A2 offenbart ein chirurgisches Werkzeugsystem mit einem auswechselbaren chirurgischen Schneidenzubehör und Aufsätzen zur Aufnahme der Werkzeuge, dabei umfasst dieses System ein Handstück, auf welchem das Schneidenzubehör und die Aufsätze lösbar befestigt sind. Eine Klemmhülse, welche in einem Bohrgehäuse an einem Ende des Handstücks angeordnet ist, befestigt das chirurgische Werkzeug an einen Motor, wobei eine Blattfeder den Zusammenhalt gewährleistet.

Die DE 199 18 638 A1 offenbart ein Anschlussstück für einen Bohrer zur Schaffung von Bohrungen in Knochgewebe, wobei im Anschlussstück eine Aufnahmeeinrichtung mit Rastelementen und eine die Rastelemente umgebende Schiebehülse, welche eine Sicherungsstellung gewährleistet, in dem sie eine Auslenkung der Rastelemente blockiert und eine Freigabestellung zulässt, vorgesehen ist.

Es ist auf dem Gebiete der Chirurgie und der Dentalmedizin seit langem bekannt, Werkzeuge in Werkzeugaufnahmen von Handstücken einzusetzen und ihnen durch die Werkzeugaufnahme die beabsichtigte Dreh-, Schwing- oder Vibrationsbewegung zu vermitteln. Dazu gibt es eine Vielzahl unterschiedlicher Systeme und eine große Anzahl von speziell ausgebildeten und an die jeweiligen Werkzeugaufnahmen angepaßten Werkzeugschäfte. Von Bedeutung ist bei der Verwendung insbesondere im chirurgischen Bereich, bei dem in vielen Fällen größere Volumina von Knochen oder anderen harten Materialien abgetragen werden müssen als im Dentalbereich, dass Bohrer oder allgemein Werkzeuge verwendet werden, die in ihrem Inneren eine Zuleitung für ein Kühlmedium aufweisen. Dies dient einem wirksamen Kühlen der Arbeitsstelle und einem geordneten Austrag des abgespanten Materials von der Bearbeitungsstelle.

Darüberhinaus werden die gleichen Handstücke und damit die gleichen Werkzeugaufnahmen auch verwendet, um in gebohrte Löcher im Knochen Gewinde zu schneiden und um Implantate in die so geschaffenen Gewinde einzuschrauben. Insbesondere beim Einschrauben der Implantate werden Drehmomente übertragen, die deutlich höher liegen als beim Bohren des Loches im Knochen oder Zahn. Größenordnungsmäßig kann gesagt werden, dass hier Drehmomente bis 80 Newtonzentimeter angewandt werden, die nun über einen Werkzeugschaft mit einem üblichen Nominaldurchmesser von 2,35 mm übertragen werden müssen. Dabei ist noch zu bedenken, dass diese Werkzeugschäfte ja in ihrem Inneren ein Längsloch aufweisen, um das Zufuhrröhrchen der Werkzeugaufnahme, durch das die Kühlflüssigkeit geleitet wird, aufnehmen zu können, so dass nur ein dünner Mantelbereich des Werkzeugschaftes zur Übertragung des Drehmomentes zur Verfügung steht.

Die heute überwiegend verwendeten, dem eingangs genannte Stand der Technik entsprechenden, Werkzeugaufnahmen übertragen dabei das Drehmoment auf folgende Weise auf den Werkzeugschaft: Der Werkzeugschaft ist an seinem oberen Ende über eine Höhe von einigen Millimetern abgeplattet ausgebildet, diese Abplattung liegt an einem entsprechend vorstehenden, flachen, Teil der Werkzeugaufnahme an und bildet so eine formschlüssige Verbindung mit der Werkzeugaufnahme. In diesem Bereich des Werkzeugschaftes ist dessen Wandstärke extrem geschwächt, da sich, axial gesehen, knapp unterhalb dieser Abplattung eine Ringnut im Werkzeugschaft befindet, durch die die axiale Sicherung des Werkzeuges in der Werkzeugaufnahme bewerkstelligt wird.

Dadurch wird der zur Verfügung stehende Hebelarm, mit dem das Drehmoment übertragen wird, extrem klein und so kommt es beim Schneiden von Gewinden und insbesondere beim Eindrehen von Implantaten häufig dazu, dass der Werkzeugschaft in diesem Bereich plastisch deformiert wird und nicht mehr aus der Werkzeugaufnahme entfernt werden kann.

Ein anderer, sehr wesentlicher Nachteil des Standes der Technik ist, dass die Abdichtung zwischen dem koaxial zur Bohrerachse ins Innere des Langloches ragenden Zufuhrröhrchens der Werkzeugaufnahme gegenüber dem drehenden Bohrer nur extrem schwer zu bewerkstelligen ist. Die heute verwendeten Lösungen sehen so aus, dass dieses still stehende Zufuhrröhrchen weit aus dem Handstück vorsteht (und somit extrem gefährdet ist, was Beschädigungen betrifft), dass der Werkzeugschaft in einem Bereich, der nahe des unteren Endes des Zufuhrröhrchens liegt (etwa in der Mitte der Werkzeuglänge!!!), eine Verdickung aufweist, dass normal zur Werkzeugachse eine Bohrung durch diese Verdickung geschaffen wird, dass im Zuge der Herstellung des Werkzeuges ein Kern, der das Zufuhrröhrchen simuliert, in die Längsbohrung des Werkzeuges eingebracht wird, und dass sodann ein Dichtmaterial (Silikon) od.dgl. durch die Querbohrung eingespritzt wird und während des Erstarrens durch Bewegen des eingeschobenen Kernes daran gehindert wird, mit diesem zu verkleben.

Nach dem Erstarren bzw. Aushärten wird der Kern entnommen und die eingespritzte Dichtmasse bildet eine völlig irregulär gestaltete Dichtung auf etwa halber Länge des Werkzeuges.

Aus all dem Gesagten geht klar hervor, dass die Schaffung eines den Anforderungen bei der Benutzung problemlos genügenden Werkzeuges und seiner Aufnahme dringend notwendig ist und dass diese neue Werkzeugaufnahme bzw. das neue Werkzeug nicht nur den Erfordernissen während der Benutzung genügen soll, sondern dass auch die Herstellungskosten so herabgesetzt werden sollen, dass es möglich ist, eine weitere Forderung der Chirurgen und Zahnärzte zu erfüllen, dass nämlich derartige Werkzeuge nach einmaligem Gebrauch weggeworfen werden, was aus Gründen der Hygiene wünschenswert ist, aus Kostengründen derzeit aber noch nicht möglich ist. Man muß dabei bedenken, dass speziell die spanenden Teile des Werkzeuges Vertiefungen, Ausnehmungen, Hohlräume u.dgl. aufweisen, die bei invasiven Operationen, die mit ihnen durchgeführt werden, organisches Material des behandelten Patienten leicht aufnehmen und nur extrem schwer wieder abgeben.

Die Erfindung löst die gestellten Aufgaben dadurch, dass der Bereich zur formschlüssigen Drehmomentübertragung durch eine polygonale Ausnehmung der Einstecköffnung der Werkzeugaufnahme gebildet ist.
Auf diese Weise wird jede Schwächung des notgedrungen schon geringen Querschnittes des Werkzeuges in dem axialen Bereich, der vom Drehmoment belastet wird, zuverlässig vermieden, während gleichzeitig der Platz für die Dichtung bei den Werkzeugen, die über eine Innenkühlung verfügen, an die Stelle gerückt wird, an der eine derartige Dichtung leicht und zuverlässig einzusetzen ist, den oberen Endbereich des Werkzeugschaftes. Werkzeuge, die über keine Innenkühlung verfügen, beispielsweise die eingangs genannten Werkzeuge zum Einschrauben von Implantaten, können in dem Bereich, in dem das Drehmoment von der Werkzeugaufnahme übertragen wird, bereits voll ausgeführt sein und benötigen nur in ihrem oberen, praktisch drehmomentfreien Bereich eine Ausnehmung, um das Zufuhrröhrchen aufnehmen zu können.

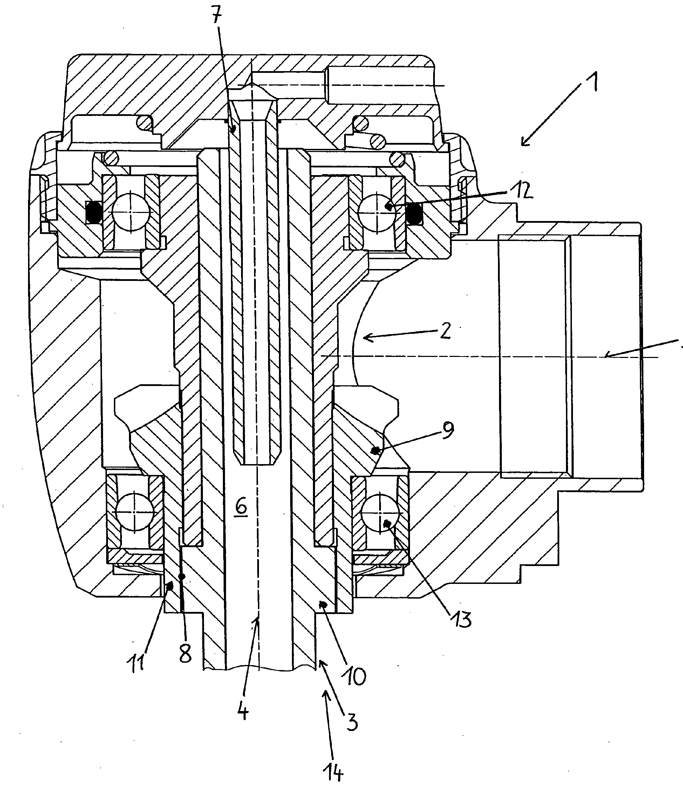

Erfindungsgemäß ist daher die Werkzeugaufnahme so ausgebildet, dass der Bereich zur formschlüssigen Drehmomentübertragung durch eine polygonale Ausnehmung der Einstecköffnung der Werkzeugaufnahme gebildet ist. In einer Ausgestaltung der Erfindung ist vorgesehen, dass das Element zur formschlüssigen Übertragung des Drehmomentes in der Werkzeugaufnahme einstückig mit dem Zahnrad ausgebildet ist, das das Drehmoment vom Antrieb überträgt. Auf diese Weise wird die beim Stand der Technik immer wieder vorkommende Verdrehung zwischen diesem Zahnrad und der eigentlichen Werkzeugaufnahme vermieden.
Die Erfindung wird im folgenden an Hand der Zeichnung, die aus nur einer Figur besteht, näher erläutert.
Die einzige Figur zeigt das vordere Ende eines erfindungsgemäßen Handstückes 1 im Schnitt entlang der Drehachse 4 des Werkzeuges 3 und der Drehachse 5 des (nicht dargestellten) Antriebes im Handstück 1.
Das Werkzeug 3, im dargestellten Fall ein Bohrer, Fräser od.dgl. mit Innenkühlung weist zur Zuleitung der Kühlflüssigkeit eine Längsbohrung 6 auf, die vom oberen Schaftende bis zu dem, nicht dargestellten, eigentlichen Arbeitsbereich führt, wo durch entsprechende Kanäle die Durchleitung der Kühlflüssigkeit durch den Werkzeugkopf zur eigentlichen Bearbeitungsstelle erfolgt. Um die Kühlflüssigkeit in die Längsbohrung 6 des Werkzeuges 3 einzubringen, ist in der Werkzeugaufnahme 2 ein Zufuhrröhrchen 7 vorgesehen, das koaxial mit dem Werkzeug 3 verläuft und dessen Abmessungen so gewählt sind, dass sein Außendurchmesser kleiner ist als der Innendurchmesser der Längsbohrung 6 und dass es, axial gesehen, in einem Bereich der Werkzeugaufnahme 2 endet, der noch oberhalb des Bereiches 8 der Drehmomentübertragung liegt.

Dieser Bereich 8 der Drehmomentübertragung liegt im untersten Abschnitt des Handstückkopfes. Mit "unten" bzw. "oben" werden in der Anmeldung und den Ansprüchen die Richtungen verstanden, wie sie beim Betrachten der Zeichnung vorliegen. In der Werkzeugaufnahme ist "unten" die Seite, von der das Werkzeug eingesteckt wird, für das Werkzeug ist "unten" die Seite, an der sich der Werkzeugkopf befindet und "oben" die Seite des Werkzeugschaftes, in die das Zufuhrröhrchen 7 gesteckt wird.

Die Drehmomentübertragung erfolgt einerseits mittels eines hülsenartigen Fortsatzes 11 des Antriebszahnrades 9, der sich bis in diesen Bereich 8 erstreckt, andererseits weist der Schaft des Werkzeuges 3 in diesem Bereich 8 eine Verdickung 10 auf, dies jeweils mit der Maßgabe, dass die Innenkontur des hülsenartigen Fortsatzes 11 mit der Außenkontur der Verdickung 10 korrespondiert und dass durch diese Übereinstimmung eine formschlüssige Verdrehsicherung geschaffen wird.

Wie aus dem Gesagten leicht entnehmbar ist und im Zusammenhang mit der Zeichnung direkt ersichtlich ist, wird das Drehmoment vom Zahnrad 9 über seinen hülsenartigen Fortsatz 11 und die formschlüssige Verbindung mit der Verdickung 10 auf den Schaft des Werkzeuges 3 übertragen, der von diesem axialen Bereich an nach unten zum (nicht dargestellten) Bearbeitungskopf hin, keinerlei Schwächung erleidet.

Wenn statt des dargestellten Werkzeuges 3 mit Innenkühlung ein Schlüssel oder Übertragungsstift od.dgl. eingespannt wird, mit dem beispielsweise ein Implantat eingeschraubt wird, so kann dieser, wie leicht ersichtlich, unmittelbar unterhalb des Bereiches, in dem das Zufuhrröhrchen 7 endet, voll ausgebildet sein, d.h., dass für den gesamten Bereich, der mit dem hohen Drehmoment für das Eindrehen beaufschlagt wird, der volle Querschnitt zur Verfügung steht. Dieser Querschnitt kann u.U. auch größer sein als der des dargestellten Werkzeuges 3, da, wie aus der Figur ersichtlich ist, vom Bereich der Drehmomentübertragung 8 an, der Werkzeugkopf 1 und die Werkzeugaufnahme 2 es erlauben, dass ein Werkzeugschaft mit größerem Durchmesser (in diesem Bereich, natürlich nicht im oberen Bereich der Werkzeugaufnahme) verwendet wird.

Wenn man auf die Verwendung von Bohrern mit dem oben genannten Standarddurchmesser von 2,35 mm verzichtet und beispielsweise auf Durchmesser von 3 mm übergeht, so kann man die Längsbohrung für die Zufuhr der Kühlflüssigkeit durch spanende Bearbeitung schaffen und ist nicht mehr auf die extrem teure Methode des Senk-Erodierens angewiesen. Die Zahl der Einsatzmöglichkeiten für derartige Bohrer nimmt bei der dadurch erzielbaren Kostenreduktion stark zu.

Die Details der Ausführung, wie die verwendbaren Materialien, die Herstellungsverfahren, die Anpassung an bestehende Systeme sind für den Fachmann in Kenntnis der Erfindung, leicht bestimmbar und brauchen hier nicht abgehandelt zu werden.

## Patentansprüche

1. Handstück für ein chirurgisches oder dentales, in den Kopf des Handstückes (1) durch eine Einstecköffnung (14) in eine Werkzeugaufnahme (2) einsteckbares, mittels zweier, axialen Abstand voneinander aufweisenden Lager (12, 13) rotierend gelagertes Werkzeug (3) mit einem im Bereich des Lagers (13), das der Einstecköffnung (14) benachbart ist, vorgesehenen Bereich (8) zur formschlüssigen Drehmomentübertragung auf das Werkzeug (3), **dadurch gekennzeichnet, dass** der Bereich (8) zur formschlüssigen Drehmomentübertragung durch eine polygonale Ausnehmung der Einstecköffnung der Werkzeugaufnahme (2) gebildet ist.

2. Handstück nach Anspruch 1, **dadurch gekennzeichnet, dass** der Bereich (8) zur formschlüssigen Drehmomentübertragung im Bereich der Einstecköffnung (14) außerhalb der beiden Lager (12, 13), angeordnet ist.

3. Handstück nach Anspruch 1 oder 2, **dadurch gekennzeichnet, dass** die polygonale Ausnehmung ein regelmäßiges Sechseck ist.

4. Handstück nach einem der Ansprüche 1 bis 3, **dadurch gekennzeichnet, dass** der Bereich (8) zur formschlüssigen Drehmomentübertragung einstückig mit einem Zahnrad (9) ausgebildet ist, das das Drehmoment vom Antrieb überträgt.

5. Handstück nach Anspruch 4, **gekennzeichnet durch** einen hülsenartigen Fortsatz (11) des Zahnrads (9), der sich bis in den Bereich (8) zur formschlüssigen Drehmomentübertragung erstreckt.

6. Handstück nach einem der vorstehenden Ansprüche, **gekennzeichnet durch** ein Werkzeug (3) mit einem Schaft, der im Abstand vom handstückseitigen Ende des Werkzeuges einen Bereich (10) zur formschlüssigen Drehmomentübertragung aufweist, wobei der Bereich (10) polygonalen Querschnitt hat.

7. Handstück nach Anspruch 6, **dadurch gekennzeichnet, dass** der polygonale Querschnitt die Form eines regelmäßigen Sechseckes aufweist.

8. Handstück nach Anspruch 6 oder 7, **dadurch gekennzeichnet, dass** der Schaft des Werkzeuges im Bereich (10) mit dem polygonalen Querschnitt eine Verdickung aufweist, dies jeweils mit der Maßgabe, dass die Innenkontur des hülsenartigen Fortsatzes (11) mit der Außenkontur der Verdickung korrespondiert, sodass durch diese Übereinstimmung eine formschlüssige Verdrehsicherung geschaffen wird.

## Claims

1. Handpiece for a surgical or dental tool, which can be inserted in the head of the handpiece (1) through an insertion hole (14) into a tool holder (2) and is rotationally supported by means of two axially separated bearings (12, 13), with positive torque transmission to the tool (3) in the area (8) of the bearing (13) which is situated adjacent the insertion hole (14), **characterized by** the fact that the area (8) of torque transmission consists of a polygonal recess of the insertion hole of the tool holder (2).

2. Handpiece in accordance with claim 1, **characterized by** the fact that the area (8) of the torque transmission occurs in the region of the insertion hole (14) beyond the two bearings.

3. Handpiece in accordance with claim 1 or 2, **characterized by** the fact that the polygonal recess is a regular hexagon.

4. Handpiece in accordance with any of claims 1 to 3, **characterized by** the fact that the area (8) of the torque transmission is formed integrally with a gear wheel (9) which transmits torque from the power unit.

5. Handpiece in accordance with claim 4, **characterized by** the fact that the gear wheel (9) has a jacket-like prolongation (11) extending up to the area (8) of the torque transmission.

6. Handpiece in accordance with any of the preceding claims, **characterized by** a tool (3) with a shaft, which has, axially separated from the end of the tool directed to the handpiece, a region (10) for the positive torque transmission, the region (10) having a polygonal cross section.

7. Handpiece in accordance with claim 5, **characterized by** the fact that the polygonal recess is a regular hexagon.

8. Handpiece in accordance with claim 6 or 7, **characterized by** the fact that the shaft of the tool has, in the region (10) with the polygonal cross section, a swelling with the requirement that the jacket-like prolongation (11) has an inner contour which corresponds to the outer contour of the swelling, by which congruence a positive anti twist protection is reached.

## Revendications

1. Poignée porte-outil pour un outil (3) chirurgical ou dentaire, qui peut être inséré dans un logement de réception d'outil (2) dans la tête de la poignée porte-outil (1) par une ouverture d'insertion (14), et est monté rotatif au moyen de deux paliers (12, 13) présentant une distance d'espacement axiale l'un de l'autre, une zone (8) prévue dans la zone du palier (13) qui est voisine de l'ouverture d'insertion (14) étant destinée à une transmission mécanique positive de couple à l'outil (3), **caractérisée en ce que** la zone (8) pour la transmission mécanique positive de couple est formée par un évidement polygonal de l'ouverture d'insertion du logement de réception d'outil (2).

2. Poignée porte-outil selon la revendication 1, **caractérisée en ce que** la zone (8) pour la transmission mécanique positive de couple est agencée dans la zone de l'ouverture d'insertion (14) à l'extérieur des deux paliers (12, 13).

3. Poignée porte-outil selon la revendication 1 ou 2, **caractérisée en ce que** l'évidement polygonal est un hexagone régulier.

4. Poignée porte-outil selon l'une quelconque des revendications 1 à 3, **caractérisée en ce que** la zone (8) pour la transmission mécanique positive de couple est réalisée d'un seul tenant avec une roue dentée (9) qui transmet le couple depuis l'entraînement.

5. Poignée porte-outil selon la revendication 4, **caractérisée par** un prolongement (11) en forme de manchon de la roue dentée (9), qui s'étend jusque dans la zone (8) pour la transmission mécanique positive de couple.

6. Poignée porte-outil selon l'une quelconque des revendications précédentes, **caractérisée par** un outil (3) comportant une queue, qui présente, à distance de l'extrémité côté poignée porte-outil de l'outil, une zone (10) pour la transmission mécanique positive de couple, la zone (10) présentant une section transversale polygonale.

7. Poignée porte-outil selon la revendication 6, **caractérisée en ce que** la section transversale polygonale présente la forme d'un hexagone régulier.

8. Poignée porte-outil selon la revendication 6 ou 7, **caractérisée en ce que** la queue de l'outil comprend dans la zone (10) présentant la section transversale polygonale un renflement, ceci respectivement de telle manière que le contour intérieur du prolongement (11) en forme de manchon correspond au contour extérieur du renflement, de sorte que cette concordance permet d'obtenir un blocage en rotation mécanique positif.
